# EUROPEAN PATENT SPECIFICATION

(11) **EP 0 751 768 B1**
(45) Date of publication and mention of the grant of the patent: **27.12.2000**
(21) Application number: 95911429.9
(22) Date of filing: 17.03.1995
(51) Int. Cl.: A61K 31/19

(54) **ANTI-VIRAL AND ANTI-CANCER AGENT**
WIRKSTOF GEGEN VIREN UND KREBS
AGENT ANTIVIRAL ET ANTICANCEREUX

(30) Priority: 17.03.1994 GB 9405292; 18.05.1994 TR 94393
(43) Date of publication of application: 08.01.1997
(73) Proprietor: Radopath Pharmaceuticals International Limited, St Peter Port Guernsey Channel Islands GY1 6AX (GB)
(72) Inventor: AYUKO, Washington Odur, 25 Sundridge Road, Birmingham B44 9NY (GB)
(74) Representative: Matthews, Derek Peter
(86) International application number: GB9500597
(87) International publication number: WO9524897

(56) References cited:
- WO-A-91/15200
- WO-A-92/14454
- WO-A-94/27584
- GB-A- 1 283 331
- DATABASE WPI Derwent Publications Ltd., London, GB; AN 86-180544 & JP,A,61 115 020 (NIPPON SHOKUBAI KAGAKU) 2 June 1986
- 'CATALOGUE HANDBOOK OF FINE CHEMICALS' 1992 , ALDRICH CHEMIE see page 293 see page 311 see page 425 see page 429 see page 544 see page F16 - page F17 see page F19
- DATABASE WPI Derwent Publications Ltd., London, GB; AN 85-320781 & JP,A,60 224 619 (ADEKA-ARGUS CHEM KK) 9 November 1985
- MOL.TOXICOL., vol.1, no.1, 1987 pages 61 - 81 G.KLOPMAN ET AL. 'A COMPUTER AUTOMATED STUDY OF THE STRUCTURE-MUTAGENICITY RELATIONSHIPS OF NON-FUSED-RING NITROARENES AND RELATED COMPOUNDS'

## Description

The present invention relates to arylating agents, in particular phenylating agents, which are suitable as therapeutic compounds, especially in the treatment of cancer and disease caused by viral infections (eg retrovirus infections such as HIV. As shown by studies reported herein, the compound of the invention shows marked anti-tumour activity and in AIDS and pre-AIDS therapy shows the capacity to regress Kaposi's sarcoma, improve CD4 count in humans, increase the level of various blood parameters in murine patient models and to achieve weight gain and general patient condition by action against HIV and/or its effects (eg opportunistic disease).

According to the invention, there is provided the compound 2-chloro-5-nitrobenzoic acid, or a salt or ester thereof, for use in the treatment of prophylaxis or therapy of neoplasm or viral infection such as HIV-infection.

Since treatment in accordance with the invention is by what is believed to be an arylating mechanism, use is typically at relatively high concentrations and, consequently, doses. Generally, the recommended dosage is 1mg/kg body weight to 30mg/kg body weight per day for 5 days weekly.

The compound of the invention may be prepared by known process techniques for preparing benzene substituted compounds. Such techniques are described in various standard texts, for example, "Organic Syntheses" 1963 Collective Volume 4, pages 364 to 366, by Harry P Schultz and published by John Wiley and Sons Inc. Post-preparation sterilization may be required, at least following formulation of the compounds into pharmaceutical composition form.

The compound of the invention may be formulated for use as pharmaceutical compositions (eg for iv, ip, oral or sc administration) comprising at least one active compound and a diluent or carrier. Thus, the invention includes a pharmaceutical composition, which composition comprises the compound according to the invention and a pharmaceutically-acceptable diluent or carrier (eg aqueous).

Such a composition may be in bulk form or, more preferably, unit dosage form. Thus, for example, the composition may be formulated as a tablet, capsule, powder, solution or suspension. Soft gel capsules may be especially convenient. The composition may be a liposomal formulation or administered in a slow sustained release delivery system.

Compositions in accordance with the invention may be prepared using the active compound defined herein in accordance with conventional pharmaceutical practice. The diluents, excipients or carriers which may be used are well known in the formulation art and the form chosen for any particular regimen will depend on the given context and the physician's choice.

Thus, for example, as illustrated below the compound of the invention may be administered in solution in sterile deionised water. Also, if necessary, solution may be facilitated using dimethyl sulphoxide (DMSO) or alternatively an alcohol, a glycol or a vegetable oil. The compound is most favourably administered in corn oil or as a solution in DMSO/sterile water. In the case of the use according to the invention of sodium salts of the active compound, the active ingredient is preferably administered as an aqueous solution.

In using the compound of the invention, dosage guidance can be taken from animal studies such as that described below. In such studies doses of from about 50mg/kg typically up to about 1000mg/kg (eg up to about 400mg/kg, conveniently about 200mg/kg or less). Thus it is to be expected that a typical dosage for humans will be from about 5mg/kg upwards (eg up to about 20mg/kg). The concentration and dose are to be sufficient to bring an arylating mechanism into play. For intravenous administration the active compound will be dissolved or dispersed in an aqueous or other injectable liquid bearing in mind the aforementioned requirements of dosage. Typically, concentration will be 250mg/ml or less. Patient tolerance was found to be of a higher order for concentrations below that threshold although concentrations of more than 250mg/ml may be used. Typically, a preferred concentration is less than 100mg/ml, with concentrations below 50mg/ml (eg 40mg/ml) being preferred. For oral administration, enteric coated tablets will often be preferred. Maximum strength will be less than 500mg and preferably 400mg or less. Thus, for example, a range of strengths for oral administration (whether in tablet or other solid form) will be 100mg, 200mg or 400mg. Patients who are subject to oral administration will generally fast for 8 hours prior to first dose with the fast continuing for 4 hours after first dose. Free access to fluid will generally be allowed during this fasting period. Intravenous treatment will generally take place by administration very slowly over a period, typically a period of approximately 20 minutes or more. Administration will typically be by catheter, for example a catheter left in situ after flashing with 2ml of saline. Of course, the catheter will in practice then be used for taking blood samples for pharmacokinetic measurements and removed prior to the patient's departure from the clinic.

As shown by the results reported in Table 6 below, 2-chloro-5-nitrobenzoic acid shows considerable anti-tumour activity in vivo. This could not be supported in vitro and it appears the compound according to the invention requires activation in the patient's liver. This compound may also be an immunomodulator.

The following animal study illustrates the remarkable activity of the compound of the invention.

### ANIMAL STUDIES

The in vivo anti-tumour responses of the compound according to the invention were assessed against the ascitic tumours, MAC15A murine colon adenocarcinoma and various solid tumour models. The MAC15A ascites tumour cells were transplanted into male NMR1 mice by ip innoculation at a cell density of 1 x 10⁵ cells in 200µl buffer (Table I). The solid tumour models included the MAC13 and MAC16 murine colon adenocarcinomas, the B16 F1 murine melanoma and the M5076 reticulum cell sarcoma.

Treatment commenced 3 days after ip transplant or, in the case of solid tumours such as MAC13 and MAC16, treatment commenced when average tumour volumes reached 40mm³.

The animals were located in both cases into groups of 5 to 8 animals.

The animals were sacrificed after 12 days or when tumours ulcerated, tumour volume exceeded 1000mm³ or loss of body weight exceeded 50%.

Except where otherwise stated, the compound used was dissolved in DMSO and diluted in sterile distilled water, at appropriate concentrations before administration in a solvent volume of 200µl. Anti-tumour responses were obtained by comparing the median survival times or tumour growth inhibition against solvent controls. The results obtained are as shown in Tables 1 to 6 below.

### Preparation

Preparation of dosage solutions is exemplified as follows:-

| | | |
|---|---|---|
| Subjects: | No | 10 animals |
| | Weight | 22g |
| Dosage: | 50mg/kg weight per animal per day thus 1.1mg per mouse per day | |
| Total Mass Dosage: | 55mg active ingredient (referred to 5 day treatment regime) | |
| Total Formulation: | 10ml solvent plus 55mg for division into 50 doses of 1.1mg dissolves in 200µl solvent | |

### T/C%

T/C% is determined as follows:-

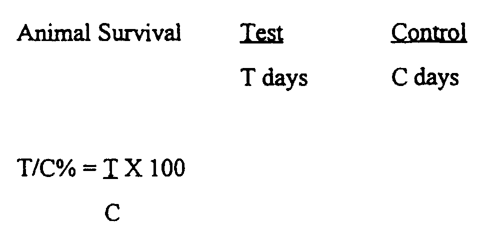

### Example

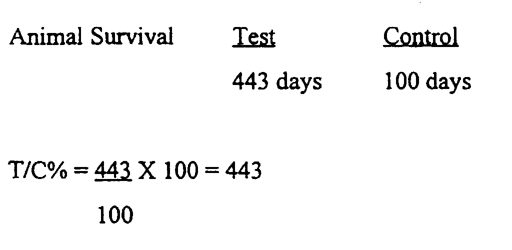

A figure of 158 or above indicates performance justifying clinical trial.

### Conclusions

The effect of a group of compounds on the growth rate of a number of experimental tumours has been evaluated in vivo and the following findings were noted:-
1. Structure-activity relationships against the MAC15A murine colon adenocarcinoma, in the female NMRI mice showed maximal activity on a split-dose schedule and when the halogen was maximally activated for nucleophilic attack.
2. Against the M5076 reticulum cell sarcoma, 2, 4-dichloro-3, 5-dinitrobenzoic acid showed activity on a split-dose schedule down to 25mg/kg body weight by both ip and sc routes. Both the amide and the methyl ester showed 10-fold increase in toxicity and were without anti-tumour activity. The acid also effectively inhibited growth of B16 murine melanoma and the MAC16 murine colon adenocarcinoma.

It is concluded that this group of compounds show a wide spectrum of activity against murine models.

**TABLE 1**

| Anti-tumour activity against MAC15A (murine adenocarcinoma colon). 5 animals per group. Dose 100mg kg⁻¹ ip per day. | | | |
|---|---|---|---|
| Code | Compound | Schedule (days) | T/C%^{a} |
| C4 | 4-chloro-3,5-dinitrobenzoic acid | 1,2,3,4,5 | 271 |
| C1 | 2,4-dichloro-3,5-dinotrobenzoic acid | 1,2 | 243 |
| C12 | 2,5-dichlorobenzoic acid | 1,2,3,4,5 | 171 |
| C28 | 2,4-dinitrobenzoic acid | 1,2,3,4,5 | 100 |
| C29 | 3,5-dinitrobenzoic acid | 1,2,3,4,5 | 100 |

| | | | |
|---|---|---|---|
| a = median T-test group, C-solvent control | | | |

**TABLE 2**

| Anti-tumour activity against M5076-reticulum cell sarcoma 16 days after im transplant. 7 animals per group. Drugs dissolved in corn oil. Dosage is per day. | | | | |
|---|---|---|---|---|
| Compound | Dose (mg/kg) | Route | Schedule (days) | % Tumour Weight Inhibition |
| 2,4 BA | 75^{a} | ip | 1,4,6,9 | 79,88^{b} |
| | 50 | ip | 1,4,6,9 | 57 |
| | 25 | ip | 1,2,4,6,9 | 75 |
| | 75 | sc | 1,4,5,7,9 | 66 |
| | 50 | sc | 1,2,4,5,6,7,9 | 76 |
| | 25 | sc | 1,2,4,5,6,7,9 | 63 |
| 2,4 BM | 1.0^{a} | ip | 1,2,3,4,5,6,7,8,9 | 41 |
| | 0.5 | ip | 1,2,3,4,5,6,7,8,9 | 39 |
| | 0.25 | ip | 1,2,3,4,5,6,7,8,9 | 42 |

| | | | | |
|---|---|---|---|---|
| a = Maximum tolerated dose | | | | |
| b = Two independent experiments; 4 animals had no tumour in the second experiment | | | | |
| 2,4 BA = 2,4-dichloro-3,5-dinitrobenzoic acid (C1) | | | | |
| 2,4 BM = 2,4-dichloro-3,5-dinitrobenzoic acid methyl ester (C3) | | | | |

% Tumour Weight Inhibition:-

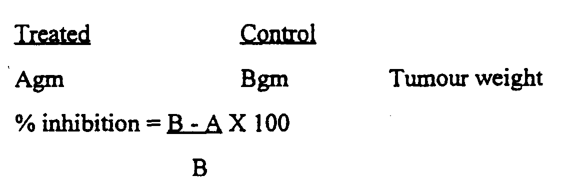

**TABLE 3**

| Anti-tumour activity against B16F1-murine melanoma 12 days after sc transplant. 6 animals per group. Drugs dissolves in corn oil. Dosage is per day. | | | | |
|---|---|---|---|---|
| Compound | Dose (mg/kg) | Route | Schedule (days) | % Tumour Weight Inhibition |
| 2,4 BA | 75^{a} | ip | 1,5 | 71,81^{b} |
| | 50 | ip | 1,5 | 45,56^{b} |
| | 25 | ip | 1,5 | 13 |
| | 75 | sc | 1,3,5 | 30 |
| | 50 | sc | 1,3,5 | 9 |
| | 25 | sc | 1,3,5 | 22 |
| 4 BA | 100 | ip | 1,5 | 39 |
| | 75 | ip | 1,5 | 41 |
| | 50 | ip | 1,5 | 10 |
| 4 BM | 2.5^{a} | ip | 1,3 | 67 |
| | 1.25 | ip | 1,2,3 | 43 |

| | | | | |
|---|---|---|---|---|
| a = Maximum tolerated dose | | | | |
| b = Two independent experiments | | | | |
| 2,4 BA = 2,4-dichloro-3,5-dinitrobenzoic acid (C1) | | | | |
| 4BA = 4-chloro-3,5-dinitrobenzoic acid (C4) | | | | |
| 4 BM = 4-chloro-3,5-dinitrobenzoic acid methyl ester (C6) | | | | |

**TABLE 4**

| Anti-tumour activity against MAC13 murine colon adenocarcinoma 12 days after im transplant. Drugs dissolved in corn oil. Dosage is per day. | | | | |
|---|---|---|---|---|
| Compound | Dose (mg/kg) | Route | Schedule (days) | % Tumour Weight inhibition |
| 2,4 BA | 75^{a} | ip | 1,4,5 | 45 |
| 2,4 BA | 50 | ip | 1,2,3,4,5,6,7,8,9 | 39 |
| 2,4 BA | graph¹ | ip | graph¹ | graph¹ |
| 2 BA | graph² | ip | graph² | graph² |
| a = Maximum tolerated dose 2,4 BA = 2,4-dichloro-3,5-dinitrobenzoic acid¹ (C1) 2 BA = 2-chloro-5-nitrobenzoic acid (C17) (1: see Figure 2 of the drawings; 2: see figure 3 of the drawings) | | | | |

**TABLE 5**

| Anti-tumour activity against MAC16, murine colon adenocarcinoma sc transplanted on day 11 after the beginning of treatment with 2,4-dichloro-3,5-dinitrobenzoic acid (2,4 BA; C1). Drug dissolved in corn oil. The tumour volumes were at least 40mm³ at the beginning of the treatment. 6 animals per group. Dosage is per day. | | | | |
|---|---|---|---|---|
| Compound | Dose (mg/kg) | Route | Schedule (days) | % Tumour Weight Inhibition |
| 2,4 BA | 75^{a} | ip | 1,2,5,8 | 88 |
| | 50 | ip | 1,2,4,5,8 | 91 |

| | | | | |
|---|---|---|---|---|
| a = Maximum tolerated dose | | | | |

**TABLE 6**

| Anti-tumour activity of C17 against B16 murine melanoma 12 days after sc transplant on female C57/black mice. 6 animals per group. Dosage is per day and is ip. | | | | |
|---|---|---|---|---|
| Code | Compound | Dose (mg/kg) | Schedule (days) | % Tumour Weight Inhibition |
| C17 | 2-chloro-5-nitrobenzoic acid | 700 | 1,2,3,4,5,6 | 62 |

Anti-tumour activity and toxicity studies have additionally been completed for the following compounds with broadly satisfactory results:-
- C22: 2,5-dichloro-4-nitrobenzoic acid
- C23: 2,4-dichloro-5-nitrobenzoic acid
- C24: 2,6-dichloro-4-nitrobenzoic acid
- C26: 2-hydroxy-5-nitrobenzoic acid
- C27: 3,5-dichloro-4-nitrobenzoic acid

In addition, the following primary assay was used to investigate the anti-viral activity of compounds.

### Primary Assay

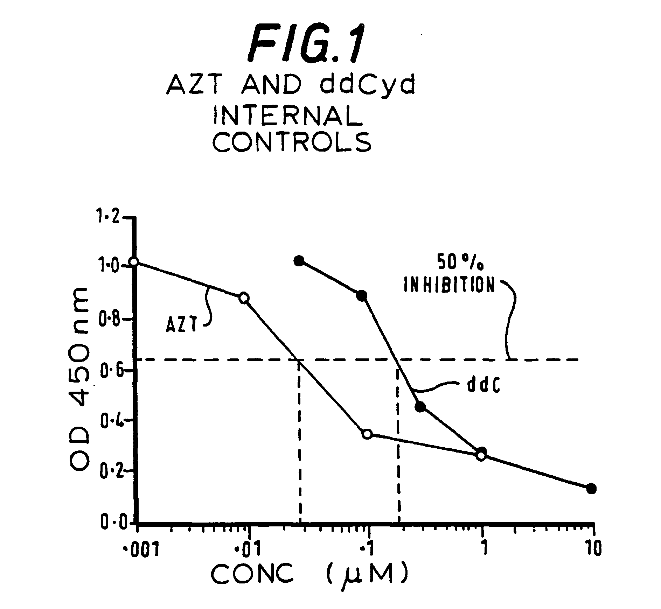

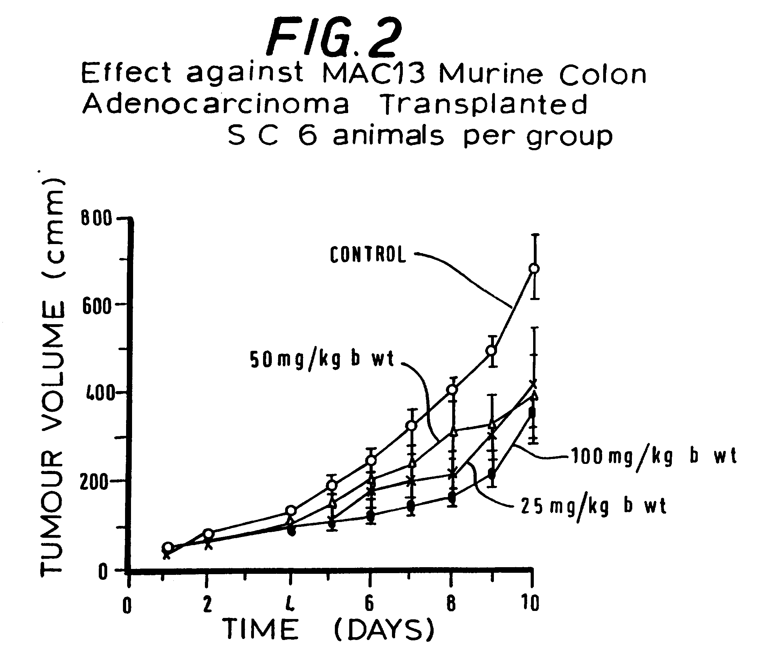

(i) *Acute Infection Assay*. High titre virus stocks of the human immunodeficiency virus HIV-1_{RF} were grown in H9 cells with RM1 1640 (Flow laboratories) supplemented with 10% fetal calf serum, penicillin (100IU/ml). Cell debris was removed by low speed centrifugation, and the supernatant stored at -70°C until required. In a typical assay C8166 T-lymphoblastoid CD4+ cells were incubated with 10xTCID50 HIV-1_{RF} at 37°C for 90 minutes and then washed three times with phosphate buffered saline (PBS). Cell aliquots (2 x 10⁵) were resuspended in 1.5ml growth medium in 6ml tubes, and active compounds in log dilutions [200µM to 0.2µM] were added immediately. 20mM stock solutions of each compound were made up in 70% alcohol. The compounds were stored as a powder and made up freshly in distilled water before each experiment or were stored as a 90 mM stock solution in 70% alcohol. The final concentration of alcohol in the tissue culture medium was 1%. The cells were then incubated at 37°C in 5% CO₂. At 72 hours post-infection 200µl of supernatant was taken from each culture and assayed for HIV (Kingchington et al, 1989, Robert et al 1990) using an antigen capture ELISA which recognizes all the core proteins equally (Coulter Electronics, Luton, UK). The following controls were used: supernatants taken from uninfected and infected cells, infected cells treated with AZT (Roche Products UK Ltd) and ddC (Roche) and RO31-8959 (Roche) an inhibitor of HIV proteinase. The IC₅₀ activities of 8959, AZT and ddC in infected cells were 1, 10, 20 nM and 200nM respectively (accompanying Figure 2). The ELISA plates were read with a spectrophotometer. Compounds were tested in duplicate at each concentration, and the data shown is the average of at least two assays. This assay assesses the activity of compounds by measuring their inhibition of HIV core antigen levels.
(ii) *Chronically Infected Cell Assay*. Chronically infected cells (H9rf) were washed three times to remove extracellular virus and incubated with the active compounds (200-0.2 µM) for four days. HIV-1 antigen in the supernatant was then measured using an ELISA.
   To test for compound toxicity, uninfected H9 cells were incubated with the compounds for four days. Supernatants were discarded and the cells resuspended in 200µl pg growth medium containing ¹⁴C protein hydrolysate. After 6 hours the cells were harvested and the ¹⁴C incorporation measured.
(iii) *Toxicity Assay*. To test for compound toxicity, aliquots of 2 x 10⁵ of uninfected cells were cultured with the compounds in the same dilutions for 72 hours. The cells were then washed with PBSA and resuspended in 200µl of growth medium containing ¹⁴C protein hydrolysate. After 12, hours the cells were harvested and the ¹⁴C incorporation measured. Uninfected, untreated cells were used as controls. Toxicity is expressed as inhibition of uptake of ¹⁴C protein hydrolysate.

The results of these assays are summarized in Table 7 below. The IC₅₀ is the drug concentration that causes a 50% reduction in HIV core antigen levels as detected by the Coulter P24 antigen assay and is determined by doubling dilutions of supernatant taken from tubes containing untreated acutely infected cells. The CD₅₀ is the concentration of drug that causes a 50% inhibition of cells as measured by ¹⁴C protein hydrolysate uptake. The therapeutic index (TI) is determined by dividing the CD₅₀ by the IC₅₀.

**TABLE 7**

| Code | Compound | IC₅₀ | CD₅₀ | TI |
|---|---|---|---|---|
| C4 | 4-chloro-3,5-dinitrobenzoic acid | 30µm | 70µm | 2.33 |

Following the same methodology, more extensive assays were performed as reported in Tables 8 below.

**TABLE 8.1**

| STRUCTURE ACTIVITY RELATIONSHIP AGAINST HIV VIRUS | |
|---|---|
| CODE | COMPOUNDS |
| C1 | 2,4-dichloro-3,5-dinitrobenzoic acid |
| C3 | 2,4-dichloro-3,5-dinitrobenzoic acid methyl ester |
| C4 | 4-chloro-3,5-dinitrobenzoic acid |
| C6 | 4-chloro-3,5-dinitrobenzoic acid methyl ester |
| C7 | 2-chloro-3,5-dinitrobenzoic acid |
| C8 | 2-chloro-3,5-dinitrobenzoic acid methyl ester |
| C9 | 4-chloro-3-nitrobenzoic acid |
| C10 | 2-chloro-4-nitrobenzoic acid |
| C11 | 3,4-dichlorobenzoic acid |
| C12 | 2,5-dichlorobenzoic acid |
| C17 | 2-chloro-5-nitrobenzoic acid |

**TABLE 8.2**

| | IC50 (Antiviral) | CC50 (Toxicity) | SI (Selectivity Index) |
|---|---|---|---|
| | | | |

| Against HIV-I IIIB | | | |
|---|---|---|---|
| C1 | 5 | 70 | 14 |
| | 36 | 70 | 2 |
| | 33 | 70 | 2 |
| | 35 | 60 | 2 |
| **Average** | **27** | **70** | **3** |
| C1 | 10 | 30 | 3 |
| | 2.5 | 20 | 8 |

| Against HIV-IRF | | | |
|---|---|---|---|
| C1 | 7 | 60 | 8.5 |
| | - | - | 56 |
| | 16 | 56 | 3.5 |
| **Average** | **11.5** | **57** | **5** |

| Against Chronically infected cells | | | |
|---|---|---|---|
| C1 | 16 | 30 | 2 |
| | 16 | 95 | 6 |
| **Average** | **16** | **63** | **4** |

| Against HIV-1-IIIB | | | |
|---|---|---|---|
| C2 | 2 | 70 | 35 |
| C3 | 0.3 | 7 | 23 |
| C4 | 40 | 100 | 2.5 |
| | 30 | 70 | 2.3 |
| **Average** | **35** | **85** | **2.4** |
| C5 | 5 | 50 | 10 |
| C6 | 5 | 60 | 12 |
| C7 | 23 | 150 | 6 |
| | 5 | >200 | >10 |
| **Average** | **22** | **>175** | **8** |
| C7 | >1 | 35 | >35 |
| | 10 | 30 | 3 |
| C8 | 10 | 60 | 5 |
| C9 | >200 | >200 | - |
| C10 | >200 | >200 | - |
| C11 | >200 | >200 | - |
| C12 | >200 | >200 | - |

| Against HIV-I IIIB | | | |
|---|---|---|---|
| C17 | >1 | >1000 | >1000 |
| | 5 | >1000 | >200 |

### Patient Tests

A pilot study was conducted for safety, pharmacokinetics and preliminary activity of C17 in patients with HIV-1 infection and HIV-related Kaposi's sarcoma. In the pilot study, 10 patients were enrolled but only 6 were eligible for evaluation. These patients were positive for serum antibody to HIV-1 as determined by both enzyme-linked immunosorbent assay (ELISA) and Western Blot. These patients had WHO Clinical Stage 2 to 3 for HIV infection and disease and fulfilled the inclusion and exclusion criteria as per the Clinical Trial Protocol. Their CD4 ranged from 0.072 to 0.812 x 10³ cells/mm³ (normal range 1.0 x 1.3 x 10³ cells/mm³). Patient 6 had AIDS-related Kaposi's sarcoma confirmed on histology.

C17 was prepared for intravenous administration under GMP (good manufacturing practice) in strength of 40 mg/ml following the method set forth below for producing a 250mg/ml solution, additional water being added at Step 7:-

### Method of Preparation

1. To 275ml water for injection add 266.4ml 4N sodium hydroxide solution and mix.
2. Add with stirring 182.5g of active compound.
3. Add additional 4N sodium hydroxide if needed to obtain solution.
4. Filter solution through a Sterivex GV 0.22µm filter.
5. Dilute to approximately 680ml with waxer for injection.
6. Adjust to pH 7.2-7.6 with hydrochloric acid.
7. Make-up to 730ml water for injection. Adjust pH if necessary.
8. Transfer solution to aseptic room and filter through Sterivex GV 0.22µm filter into injection vials. Seal with rubber stoppers and aluminium closures.
9. Effect terminal sterilization by autoclave.

The patients received 10mg/kg body weight/day of C17 by deep intramuscular injection daily for 5 days weekly. The CD4 counts were estimated before and at about 2 weeks after the beginning of the therapy.

The CD4 counts in all 6 patients showed an increase which was associated with clinical improvements in patient general condition including weight gain and a marked decrease in opportunistic infections and diarrhea. Patient observations are shown in Tables 9 below.

In the case of Patient 6, the sarcoma lesions disappeared.

### Murine Haematology

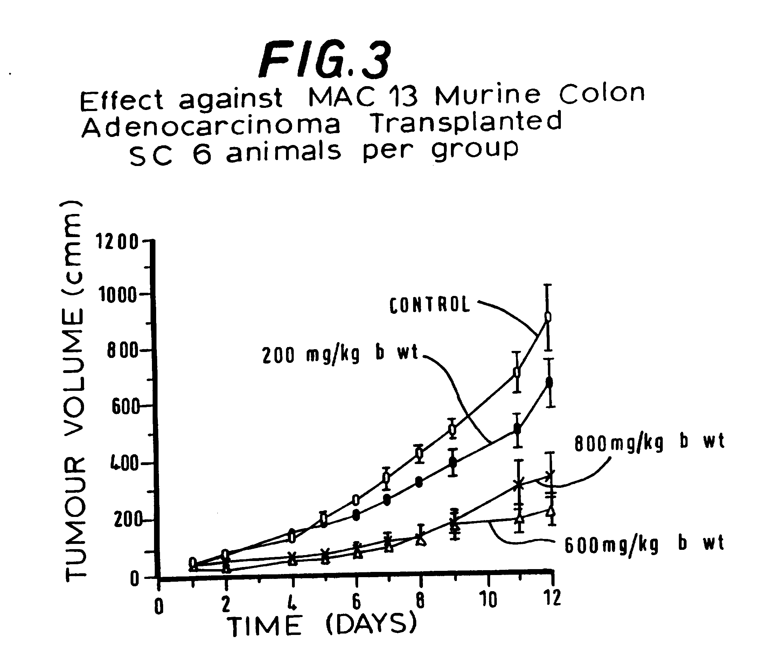

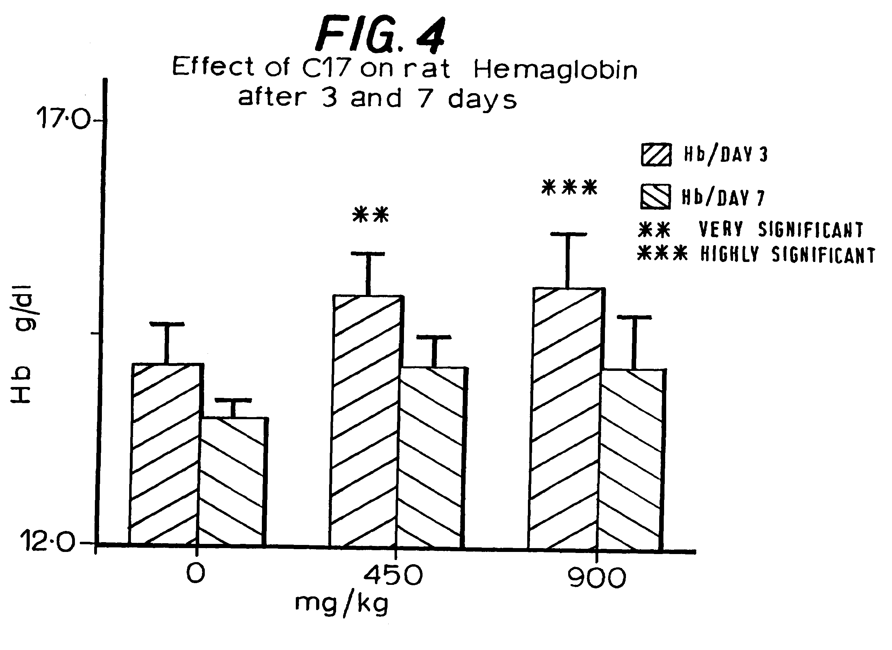

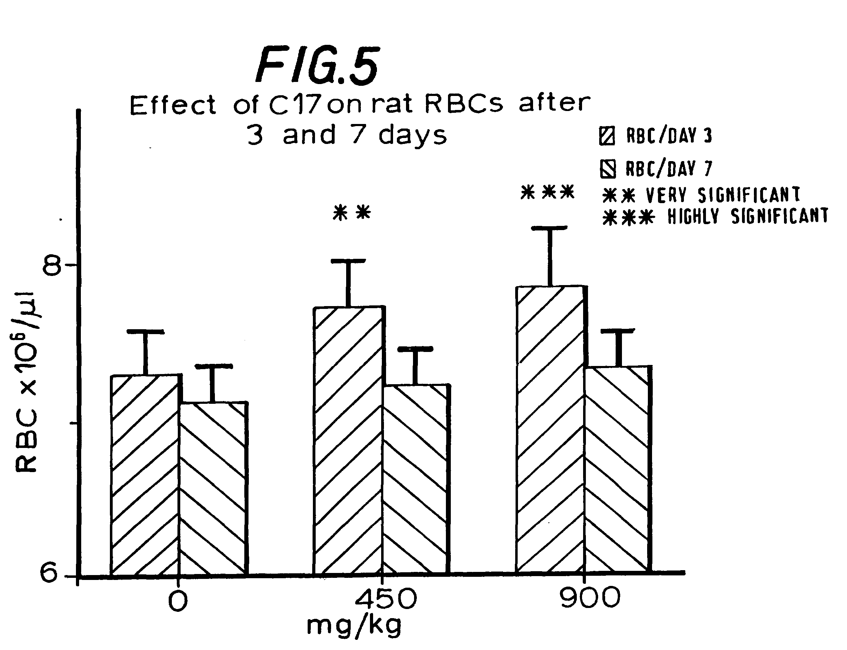

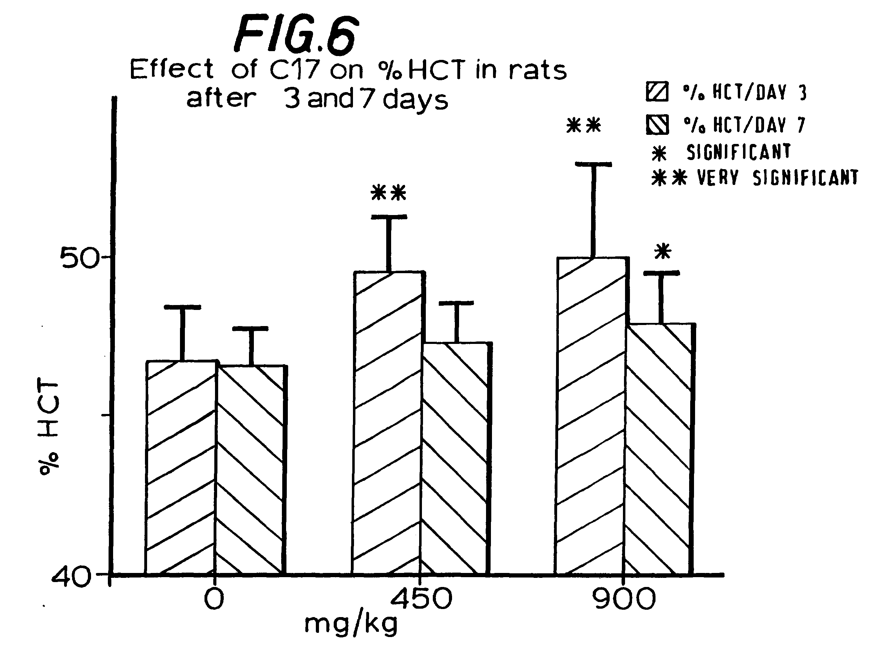

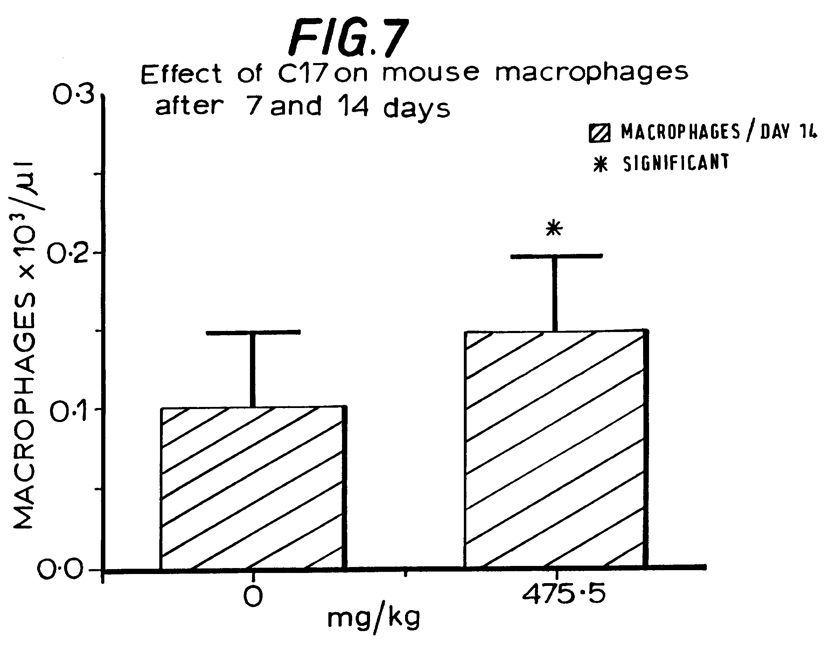

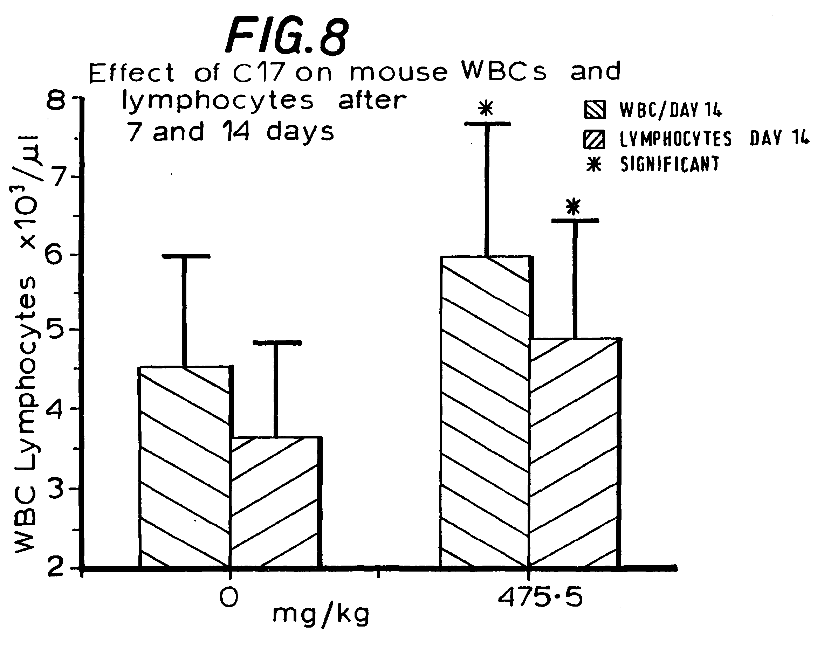

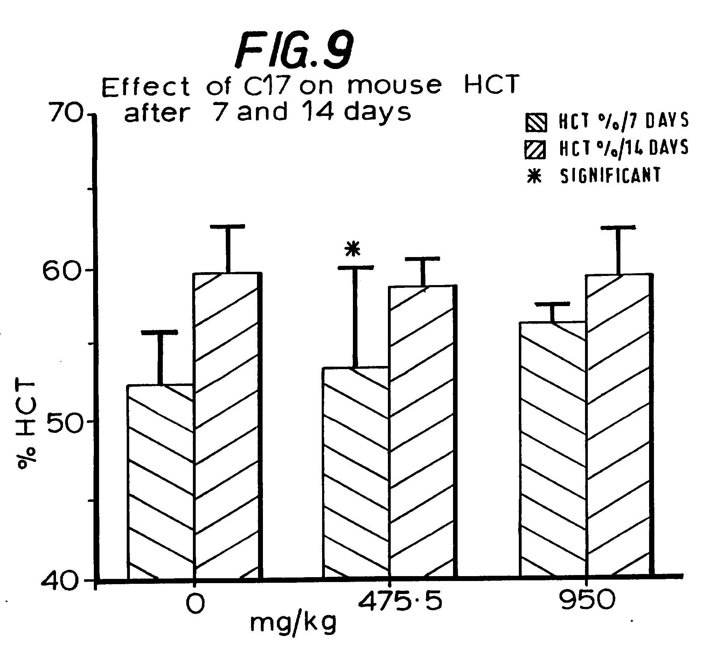

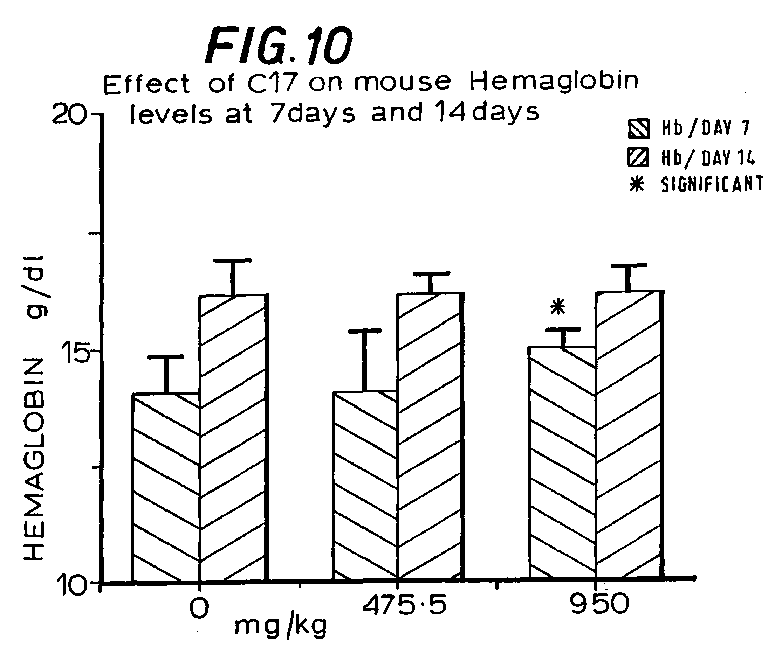

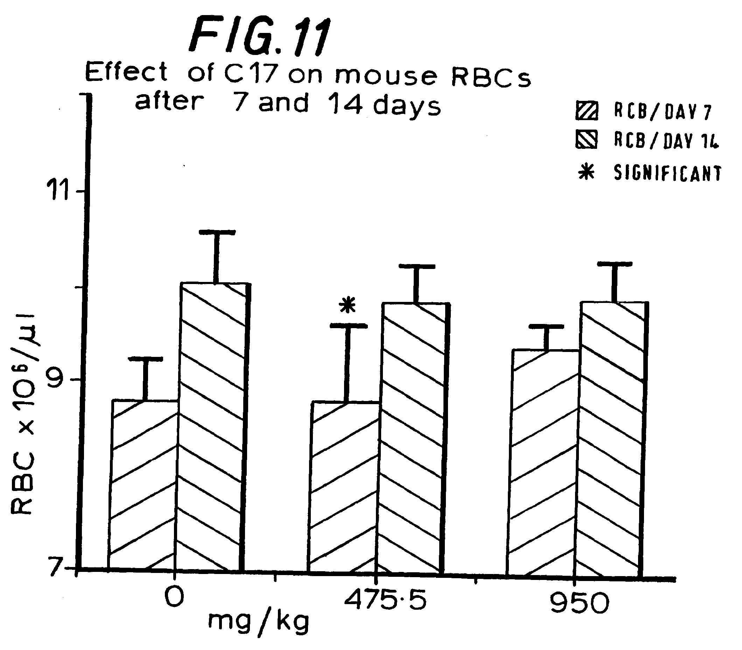

Preliminary data on the administration of C17 in mice and rats showed increases in red blood cell count, haemoglobin concentration, macrophage count, white blood cell count (WBC), lymphocyte count and haematocrit (HTC) on day 7 when maximum tolerated dose (MTD) was given by 5 consecutive daily intravenous injections. These values returned to normal on day 14. The MTD values were 950mg and 900mg/kg body weight/day respectively in mouse and rat. The data are reported in Figures 4 to 11.

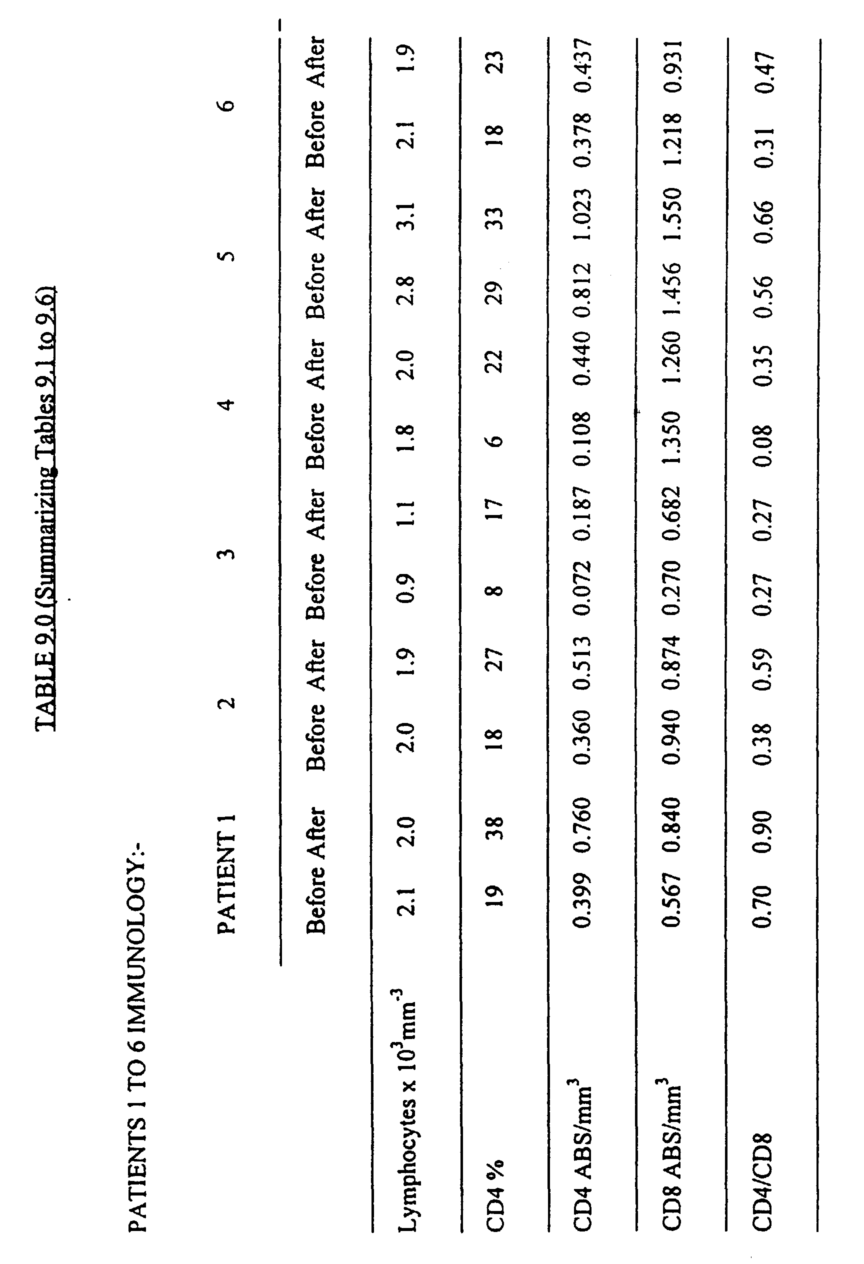

**TABLE 9.1**

| PATIENT 1 IMMUNOLOGY:- | | |
|---|---|---|
| DAY | 1 | 20 |
| Lymphocytes x 10³mm⁻³ | 2.1 | 2.0 |
| CD4 % | 19 | 38 |
| CD4 ABS/mm³ | 0.399 | 0.760 |
| CD8 ABS/mm³ | 0.567 | 0.840 |
| CD4/CD8 | 0.70 | 0.90 |

**TABLE 9.2**

| PATIENT 2 IMMUNOLOGY:- | | |
|---|---|---|
| DAY | 1 | 14 |
| Lymphocytes x 10³mm⁻³ | 2.0 | 1.9 |
| CD4 % | 18 | 27 |
| CD4 ABS/mm³ | 0.360 | 0.513 |
| CD8 ABS/mm³ | 0.940 | 0.874 |
| CD4/CD8 | 0.38 | 0.59 |

**TABLE 9.3**

| PATIENT 3 IMMUNOLOGY:- | | |
|---|---|---|
| DAY | 1 | 20 |
| Lymphocytes x 10³mm⁻³ | 0.9 | 1.1 |
| CD4 % | 8 | 17 |
| CD4 ABS/mm³ | 0.072 | 0.187 |
| CD8 ABS/mm³ | 0.270 | 0.682 |
| CD4/CD8 | 0.27 | 0.27 |

**TABLE 9.4**

| PATIENT 4 IMMUNOLOGY:- | | |
|---|---|---|
| DAY | 1 | 14 |
| Lymphocytes x 10³mm⁻³ | 1.8 | 2.0 |
| CD4 % | 6 | 22 |
| CD4 ABS/mm³ | 0.108 | 0.440 |
| CD8 ABS/mm³ | 1.350 | 1.260 |
| CD4/CD8 | 0.08 | 0.35 |

**TABLE 9.5**

| PATIENT 5 IMMUNOLOGY:- | | |
|---|---|---|
| DAY | 1 | 21 |
| Lymphocytes x 10³mm⁻³ | 2.8 | 3.1 |
| CD4 % | 29 | 33 |
| CD4 ABS/mm³ | 0.812 | 1.023 |
| CD8 ABS/mm³ | 1.456 | 1.550 |
| CD4/CD8 | 0.56 | 0.66 |

**TABLE 9.6**

| PATIENT 6 IMMUNOLOGY:- | | |
|---|---|---|
| DAY | 1 | 34 |
| Lymphocytes x 10³mm⁻³ | 2.1 | 1.9 |
| CD4 % | 18 | 23 |
| CD4 ABS/mm³ | 0.378 | 0.437 |
| CD8 ABS/mm³ | 1.218 | 0.931 |
| CD4/CD8 | 0.31 | 0.47 |

## Claims

1. 2-Chloro-5-nitrobenzoic acid, or a salt or ester thereof, for use as a pharmaceutical.

2. A compound as claimed in Claim 1 for use in the treatment by prophylaxis or therapy of neoplasm or viral infection.

3. A pharmaceutical composition for use in the treatment by prophylaxis or therapy of neoplasm or viral infection, the composition comprising 2-chloro-5-nitrobenzoic acid or a salt or ester thereof, in combination with a pharmaceutically-acceptable carrier or diluent.

4. A composition as claimed in Claim 3 which is in tablet, capsule, powder, solution or suspension form.

5. A composition as claimed in Claim 3 or Claim 4 which is in the form of an injectable solution or dispersion in an aqueous or other injectable liquid carrier in a concentration of less than 250 mg/ml.

6. A composition as claimed in any one of Claims 3 to 5 wherein the carrier or diluent is aqueous.

7. A composition as claimed in Claim 3 or Claim 4 wherein the carrier is a solid excipient.

8. A composition as claimed in Claim 7 which is in the form of enteric coated tablets comprising 2-chloro-5-nitrobenzoic acid, or a salt or ester thereof, in strength 100 to 400 mg, the composition being formulated as a mixture comprising the compound together with an inert pharmaceutically-acceptable solid tabletting carrier.

9. A composition as claimed in any one of Claims 3 to 8 which is in unit dosage form.

10. A composition as claimed in any one of Claims 3 to 9 and comprising the 2-chloro-5-nitrobenzoic acid, or a salt or ester thereof, encapsulated by a soft gel capsule or contained by a sustained release drug delivery system.

11. A method of preparing a pharmaceutical composition as claimed in any one of Claims 3 to 10 for use in the treatment by prophylaxis or therapy of neoplasm or viral infection, which method comprises combining 2-chloro-5-nitrobenzoic acid, or a salt or ester thereof, with aqueous sodium hydroxide solution to form a solution, adjusting the pH of the solution to an alkaline pH of not more than 8 by addition to the solution of an acidic material, optionally diluting the pH-adjusted solution with physiologically injectable water and sterilizing the solution.

12. Use of 2-chloro-5-nitrobenzoic acid or a salt or ester thereof, for the preparation of a medicament for the treatment by prophylaxis or therapy of neoplasm or viral infection in mammalian subjects.

13. Use as claimed in Claim 12 wherein the viral infection is HIV-related Kaposi's sarcoma.

## Patentansprüche

1. 2-Chlor-5-Nitrobenzoesäure oder ein Salz oder ein Ester hiervon, zur Verwendung als Pharmazeutikum.

2. Verbindung nach Anspruch 1 zur Verwendung bei der prophylaktischen oder therapeutischen Behandlung von Neoplasmen oder Virusinfektionen.

3. Pharmazeutische Zusammensetzung zur prophylaktischen oder therapeutischen Behandlung von Neoplasmen oder Virusinfektionen, enthaltend 2-Chlor-5-Nitrobenzoesäure oder ein Salz oder einen Ester hiervon in Kombination mit einem pharmazeutisch akzeptablen Träger oder Verdünnungsmittel.

4. Zusammensetzung nach Anspruch 3, welche in Form von Tabletten, Kapseln, Pulver, eine Lösung oder einer Suspension vorliegt.

5. Zusammensetzung nach einem der Ansprüche 3 oder 4, welche in Form einer Injektionslösung, oder injizierbaren Dispersion in einem wässrigen oder einem anderen injizierbaren flüssigen Träger mit einer Konzentration von weniger als 250 mg/ml vorliegt.

6. Zusammensetzung nach einem der Ansprüche 3 bis 5, wobei der Träger oder das Verdünnungsmittel wässrig sind.

7. Zusammensetzung nach einem der Ansprüche 3 oder 4, worin der Träger ein fester Arzneimittelträger ist.

8. Zusammensetzung nach Anspruch 7, welche in Form von enteral beschichteten Tabletten, enthaltend 2-Chlor-5-Nitrobenzoesäure oder ein Salz oder einen Ester hiervon in einer Menge von 100 - 400 mg, vorliegt und wobei die Zusammensetzung als eine Mischung enthaltend die Verbindung zusammen mit einem inerten, pharmazeutisch akzeptablen festen Tablettierträger formuliert ist.

9. Zusammensetzung nach einem der Ansprüche 3 bis 8, welche in Einheitsdosisform vorliegt.

10. Zusammensetzung nach einem der Ansprüche 3 bis 9, enthaltend 2-Chlor-5-Nitrobenzoesäure oder ein Salz oder einen Ester hiervon, welches in einer Softgel-Kapsel eingekapselt oder in einem Retardsystem zur verlangsamten Freisetzung von Wirkstoffen enthalten ist.

11. Verfahren zur Herstellung einer pharmazeutischen Zusammensetzung nach einem der Ansprüche 3 bis 10 zur Verwendung bei der prophylaktischen oder therapeutischen Behandlung von Neoplasmen oder Virusinfektionen, umfassend das Zusammenführen von 2-Chlor-5-Nitrobenzoesäure, einem Salz oder einem Ester hiervon mit einer wässrigen Natriumhydroxidlösung zur Bildung einer Lösung, Einstellen des pH-Wertes der Lösung auf einen alkalischen pH-Wert von nicht mehr als 8 durch Zusetzen eines sauren Materials zu der Lösung, gegebenenfalls Verdünnen der auf den pH-Wert eingestellten Lösung mit physiologisch injizierbarem Wasser und Sterilisieren der Lösung.

12. Verwendung von 2-Chlor-5-Nitrobenzoesäure oder eines Salzes oder eines Esters hiervon für die Herstellung eines Medikaments zur prophylaktischen oder therapeutischen Behandlung von Neoplasmen oder Virusinfektionen in Säugetieren.

13. Verwendung nach Anspruch 12 wobei die Virusinfektion ein HIV-assoziiertes Kaposi-Sarkom ist.

## Revendications

1. L'acide 2-chloro-5-nitrobenzoïque, ou un sel ou un ester dudit acide, pour une utilisation en tant que produit pharmaceutique.

2. Composé selon la revendication 1 pour une utilisation dans le traitement par prophylaxie ou thérapie d'un néoplasme ou d'une infection virale.

3. Composition pharmaceutique pour utilisation dans le traitement par prophylaxie ou thérapie d'un néoplasme ou d'une infection virale, ladite composition comprenant l'acide 2-chloro-5-nitrobenzoïque, ou un sel ou un ester dudit acide, en combinaison avec un support ou un diluant pharmaceutiquement acceptable.

4. Composition selon la revendication 3, caractérisée en ce que ladite composition se présente sous forme de pastille, capsule, poudre, solution ou suspension.

5. Composition selon la revendication 3 ou la revendication 4, caractérisée en ce que ladite composition se présente sous forme d'une solution injectable, d'une dispersion aqueuse injectable ou d'un autre support liquide injectable, à une concentration de moins de 250 mg/ml.

6. Composition selon l'une quelconque des revendications 3 à 5, caractérisée en ce que le support ou le diluant est aqueux.

7. Composition selon la revendication 3 ou la revendication 4, caractérisé en ce que le support est un excipient solide.

8. Composition selon la revendication 7, caractérisée en ce que la composition se présente sous la forme de pastilles entériques enrobées comprenant l'acide 2-chloro-5-nitrobenzoïque, ou un sel ou un ester dudit acide, en une teneur allant de 100 à 400 mg, la composition étant formulée comme un mélange comprenant l'association dudit composé et d'un support solide pharmaceutiquement acceptable destiné à une formulation sous forme de pastille.

9. Composition selon l'une quelconque des revendications 3 à 8, caractérisée en ce que la composition se présente sous forme de dose unitaire.

10. Composition selon l'une quelconque des revendications 3 à 9 et comprenant l'acide 2-chloro-5-nitrobenzoïque, ou un sel ou un ester dudit acide, encapsulé dans une capsule de gel souple ou contenu dans un système de délivrance de médicament à libération prolongée.

11. Méthode de préparation d'une composition pharmaceutique selon l'une quelconque des revendications 3 à 10 pour une utilisation dans le traitement par prophylaxie ou thérapie d'un néoplasme ou d'une infection virale, ladite méthode comprenant une combinaison de l'acide 2-chloro-5-nitrobenzoïque, ou, d'un sel ou d'un ester dudit acide, avec une solution aqueuse d'hydroxyde de sodium, de façon à former une solution, un ajustage du pH de la solution à un pH alcalin ne dépassant pas 8 par addition à la solution d'un matériel acide, éventuellement une dilution de la solution de pH ajusté avec de l'eau physiologiquement injectable et une stérilisation de la solution.

12. Utilisation de l'acide 2-chloro-5-nitrobenzoïque, ou d'un sel ou d'un ester dudit acide par la préparation d'un médicament pour le traitement par prophylaxie ou thérapie d'un néoplasme ou d'une infection virale chez des sujets mammifères.

13. Utilisation selon la revendication 12, caractérisée en ce que l'infection virale est un sarcome de Kaposi associé au VIH.
